# EUROPEAN PATENT APPLICATION

(11) **EP 2 481 747 A1**
(43) Date of publication of application: **01.08.2012**
(21) Application number: 12165603.7
(22) Date of filing: 21.07.2004
(51) Int. Cl.: C07K 1/00, A61K 31/498, A61P 5/24, A61K 9/00

(54) **Methods of treating cutaneous flushing using selective alpha-2-adrenergic receptor agonists**

(30) Priority: 23.07.2003 US 626037
(62) Divisional of application: 04778941.7
(71) Applicant: Galderma Pharma S.A., 1000 Lausanne 30 Grey (CH)
(72) Inventor: Scherer, Warren J., Naples, FL 34119 (US)
(74) Representative: Jansen, Cornelis Marinus

(57) **Abstract**

A composition comprising: at least one selective α₂ adrenergic receptor agonist selected from the group consisting of guanabenz, guanfacine, alpha-methyl DOPA (methydopamine), amphetamine, methylphenidate, lofexidine, moxonidine, dexmedetomidine, mivazerol, and (2-imidazolin-2-ylamino) quinoxaline derivatives, which derivates are formed with an acidic group other than tartrate; and a dermatologically acceptable carrier,
for use in treating cutaneous flushing in an individual, wherein the composition is to be administered to said individual via topical dermatological application in an amount sufficient to prevent, reduce, ameliorate or inhibit, cutaneous flushing.

## Description

### Cross Reference to Related Application

This application is a continuation of U.S. Application Serial No. 10/626,037, filed July 23, 2003, which is hereby incorporated by reference in its entirety, including all formulae and references.

### Field of the Invention

The present invention relates to a method of treating, reducing, inhibiting, preventing and/or reversing cutaneous facial flushing caused by abnormal, endogenously-induced vasomotor instability associated with, but not limited to acne rosacea, menopause-associated hot flashes, hot flashes resulting from orchiectomy or ingestion of substances capable of inducing a cutaneous facial flushing reaction (e.g., alcohol, chocolate, spices) by topical dermatological application of an effective dose of a composition comprising at least one α₂ adrenergic receptor agonist (such as a (2-imidazolin-2-ylamino) quinoxaline derivative such as brimonidine tartrate) and a suitable carrier.

### Background of the Invention

Facial flushing is a symptom observed in medical conditions associated with vasomotor instability. Cutaneous vasomotor instability is the term commonly used in the medical arts to refer to involuntary dilatation and reactivity of subcutaneous blood vessels. The mechanism of facial flushing involves involuntary dilation of subcutaneous arteries. The etiology underlying the initiation of facial flushing is unknown. There are essentially four common medical conditions addressed by the instant disclosure in which facial flushing occurs. These include: 1.) acne rosacea, 2.) postmenopausal hot flashes, 3.) patients who are status post surgical orchiectomy, and 4.) flushing secondary to ingestion of food substances.

Acne rosacea is a chronic dermatological disease of unknown cause characterized by facial flushing, erythema, recurrent papules and pustules, superficial telangiectasias (dilations of previously existing small blood vessels) and rhinophymia (hypertrophy of the nose with follicular dilation). The disorder is found mainly in fair-skinned patients between 30 and 50 years of age. Women are more commonly affected than men and acne rosacea is a common disorder. Because these clinical signs, rosacea was originally thought to resemble the acne (acne vulgaris) typically encountered in teenagers, however, rosacea is now known to represent a separate and distinct dermatological condition, It is estimated that approximately 13 million Americans have acne rosacea. Alcohol, stress, spicy foods and temperature extremes may exacerbate the condition.

Facial flushing associated with rosacea is due to vasomotor instability of unknown etiology. Therefore, treatments that stabilize the contractile state of cutaneous blood vessels would have a beneficial effect on this symptom. Improvement and stabilization of vascular tone via a vasoconstrictive mechanism is the approach taken by the instant disclosure. It appears that there are only two prior art patent methods of influencing vascular tone and reducing facial flushing. These include topical application of phytosphingosine (U.S. Published Application No. 2003/0068343) and nitric oxide synthetase inhibitors (WO 98/36730). Mechanistically, these differ greatly from the instant disclosure. Sphingosines are lipids that induce vasoconstriction in certain tissues via activity of specific cellular sphingosine receptors. Nitric oxide is a potent regulatory vasodilator that is produced by vascular endothelial cells. Inhibition of the enzyme that produces nitric oxide would therefore be expected to result in baseline vasoconstriction. However, the safety and tolerability of these compounds have not been established. Attempted treatment methods for facial flushing that have been published in the peer-reviewed medical literature have been limited to oral administration of the antihypertensive medication, clonidine (Guarrera *et al.,* 1982; Wilkin, 1983).

Clonidine is an alpha (α) adrenergic receptor agonist that crosses the blood-brain barrier and acts directly on the central nervous system. The chemical name of clonidine is N-(2,6-dichlorophenyl)-4,5-dihydro-1H-imidazol-2-amine. Clonidine has affinity for both the α₁ and α₂ subtypes of alpha adrenergic receptors. Traditionally, clonidine has been used to treat uncontrolled hypertension. Clonidine stimulates alpha adrenergic receptors in the brainstem, resulting in reduced sympathetic outflow that decreases renal vascular resistance, heart rate and blood pressure. Because clonidine acts directly on the central nervous system, its use is associated with multiple systemic side effects, such as bradycardia, heart block, hypotension, dizziness, dry mouth and depression. Some of the side effects may be life threatening.

For the purpose of patient convenience and desire to maintain adequate blood levels of the drug to control hypertension, clonidine has been administered via transdermal patch (U.S. Patent No. 4,201,211). However, this transdermal delivery system for clonidine is simply an alternate route of administration and does not alter its ability to affect the central nervous system or its mixed α₁ and α₂ adrenergic receptor kinetics. Topical clonidine has also been proposed to aid in alleviating neuropathic pain syndromes such as diabetic neuropathy and post-herpetic neuralgia (U.S. Patent No. 6,534,048). However, the mechanism of this analgesic action may be secondary to the release of endogenous enkephalin-like substances by the central nervous system (Nakamura *et al.,* 1988).

Research has shown that vasoconstriction of small, distal subcutaneous resistance arteries depends entirely on α₂ adrenergic receptor stimulation (Chotani *et al.,* 2000; Nielson *et al.,* 1989). Unfortunately, because of its mixed α₁ and α₂ activity, oral dosages of clonidine sufficient to produce peripheral cutaneous vasoconstriction via α₂ adrenergic receptor stimulation would also result in intolerable systemic side effects. Hot flashes are sudden sensations of flushing and heat that some women experience when they are going through menopause. Although their etiology is not completely understood, it is thought that a decrease in the female hormone estrogen leads to vasomotor instability. Symptoms include redness and warmth of the skin of the face, neck and shoulders, pounding heartbeat and sweating. Hot flashes last from a few minutes to a half hour. Approximately 20% of women seek medical treatment for postmenopausal symptoms associated with vasomotor instability. Similar symptoms are experienced by men with prostate cancer who undergo orchiectomy (surgical removal of the testes). Treatment of hot flashes requires oral estrogen replacement therapy which is thought to raise the core body temperature sweating threshold (Freedman *et al.,* 2002). However, many patients have relative or absolute contraindications to estrogen replacement therapy (*e.g.,* history of breast cancer). These patients would benefit from a safe, locally-acting compound that alleviates facial flushing. Although the exact etiology of hot flashes is unknown, the underlying physiological mechanism of facial flushing (dilation of subcutaneous arteries) is similar to that observed with rosacea in that there is endogenously-induced vasomotor instability. Treatment of the reactive cutaneous vascular bed with the (2-imidazolin-2-ylamino) quinoxaline derivative brimonidine tartrate would stimulate α₂ adrenergic receptors, thus restoring vascular tone and reversing or preventing the cutaneous flushing reaction.

It is known that patients with vasomotor instability are also susceptible to facial flushing following the ingestion of certain foods such as alcohol, chocolate, caffeine or spices. Flushing associated with ingested substances is primarily limited to the "blush area" of the mid face (Wilkin, 1988).

Topical brimonidine tartrate eye drops have been FDA approved for the treatment of elevated intraocular pressure. In addition to treating elevated intraocular pressure, brimonidine tartrate eye drops have also been patented for treating neural injury secondary to glaucoma, retinitis pigmentosa and age related macular degeneration (U.S. Patent No. 6,194,415). Brimonidine tatrate is known to have 10 fold more α₂ adrenergic receptor activity than clonidine (Burke *et al.,* 1996) and because of its hydrophilic composition, is capable of acting locally and is unable to cross the blood-brain barrier and therefore, unable to directly influence the central nervous system (Chien *et al.,* 1990). Toxicity studies have proven brimonidine tartrate to be nontoxic and to have no oncogenic or teratogenic activity (Walters, 1996).

### Brief Summary of the Invention

The instant invention involves topical cutaneous application of at least one α₂ adrenergic receptor agonist, such as the (2-imidazolin-2-ylamino) quinoxaline derivative brimonidine tartrate, which is a highly effective treatment for cutaneous facial flushing caused by vasomotor instability.

One objective of the present invention involves local cutaneous application of an effective amount of at least one α₂ adrenergic receptor agonist with an ability to act locally and inability to cross the blood-brain barrier to treat facial flushing reactions caused by vasomotor instability. In certain preferred embodiments, the α₂ adrenergic receptor agonist is bromonidine tartrate.

Thus, the instant disclosure describes a method of safely treating facial flushing in humans. This method comprises administering a composition comprising an effective amount of at least one α₂ adrenergic receptor agonist, for example, brimonidine tartrate, wherein the α₂ adrenergic receptor agonist is admixed with a dermatologically acceptable carrier or a pharmaceutically acceptable carrier. This compound, due to its properties as a highly specific, locally-acting α₂ adrenergic receptor agonist, acts to reduce cutaneous flushing via vasoconstriction of subcutaneous arteries.

### Detailed Disclosure of the Invention

The subject invention provides methods for the treatment of flushing in an individual comprising the administration of a composition comprising at least one selective α₂ adrenergic receptor agonist and a carrier in an amount sufficient to prevent, reduce, ameliorate, or inhibit facial flushing. In a preferred embodiment, brimonidine tartrate is an α₂ adrenergic receptor agonist used in the formulation of the compositions used in the subject invention. In various aspects of the subject invention, the individual is a human. In other embodiments, the subject invention treats facial flushing in individuals or humans.

Selective α₂ adrenergic receptor agomsts suitable for use in the subject invention include, and are not limited to, guanabenz, guaniacine, alpha-methyl DOPA (methydopamine), amphetamine, metllylphenidate, loflexidine, moxonidine, dexmedetomidine, mivazerol, (2-imidazolin-2-ylamino) quinoxaline derivatives (including, but not limited to, brimonidine tartrate). Brimonidine tartrate is a quinoxaline derivative and quinoxaline derivatives having α₂ receptor agonist activity were originally suggested as therapeutic agents by U.S. Patent No. 4,029,792 which is hereby incorporated by reference in its entirety.

The phrase "selective α₂ adrenergic receptor agonist(s)" is intended to convey an agonist (or agonists) that are more selective for the α₂ adrenergic receptor as compared to the α₁ adrenergic receptor. In certain embodiments of the invention, "selective α₂ adrenergic receptor agonist(s)" are at least ten (10) to 1000-fold (or higher) more selective for the α₂ adrenergic receptor than the α₁ receptor. Other embodiments provide for "selective α₂ adrenergic receptor agonist(s)" that are at least two-fold to 50-fold (or higher) more selective for the α₂ adrenergic receptor as compared to clonidine (for example, brimonidine is 7-12 fold more selective for the α₂ adrenergic receptor than is clonidine).

For the treatment of facial flushing in humans, one embodiment of the subject invention provides a (2-imidazolin-2-ylamino) quinoxaline derivative, such as brimonidine tartrate admixed with a dermatologically acceptable carrier which is then administered topically in accordance with the present invention to skin. Any suitable, conventional, dermatologically acceptable carrier may be employed. A carrier is dermatologically acceptable if it does not inhibit the effectiveness of the active compound(s) and it has substantially no long term or permanent detrimental effect on the skin to which it is administered. In various preferred embodiments, compositions of the subject invention are topically administered to facial skin.

The compositions encompassed by this invention include formulations for topical application to the human skin. For topical application, one or more α₂ adrenergic receptor agonists, such as (2-imidazolin-2-ylwnino) quinoxaline derivatives (a non-Iimitmg example of which is brimonidine tartrate), may be formulated into any pharmaceutical form normalcy employed for such an application, in particular in the form of aqueous, aqueous/alcoholic or oily solutions, dispersions of lotion or serum type, aqueous anhydrous or lipophilic gels, emulsions of liquid or semi-liquid consistency of the milk type, obtained by dispersion of a fatty phase in an aqueous phase or conversely an aqueous phase in a fatty phase, or suspensions or emulsions of semi-sofid or solid consistency of the cream or gel type, soaps or detergents, or alternatively microemulsions, microcapsules, microparticles, or vesicle dispersions of and/or non-ionic type. Among additional alternative means for topical application of compositions according to the subject invention are spray pumps, aerosol dispersions, impregnated cosmetic facial masks, and impregnated cosmetic facial cloths or sponges. These formulations may be produced by conventional techniques.

Preparation of the compositions comprising one or more α₂ adrenergic receptor agonists, such as the (2-imidazolin-2-ylamino) quinoxaline derivative brimonidine tartrate, admixed with dermatologically accepted carriers would also include standard acids, bases and buffers including, but not limited to substances such as sodium hydroxide and lactic acid to adjust and optimize pH between approximately 6.0 and 8.5.

Compositions of the subject invention can also further comprise standard dermatological preservatives to prevent the growth of microorganisms. Such standard preservatives include substances such as benzoic acid, benzyl alcohol, phenoxyethanol and parabens. Appropriate binding agents or other substances may be included to alter the viscosity or color of the final preparation.

Compositions provided by the subject invention can also contain, in addition to the components discussed *supra,* compounds known to be beneficial to the treatment of acne rosacea in addition to one or more α₂ adrenergic receptor agonists, such as the (2-imidazolin-2-ylamino) quinoxaline derivative brimonidine tartrate. These additional compounds include, and are not limited to, antibacterial agents, anthelmintic agents, antiangiogenesis agents, steroidal anti-inflammatory agents, non-steroidal anti-inflammatory agents, antioxidants or derivatives of retinoic acid, as well as halogens. Compositions according to the subject invention can also further comprise aloe for skin protection a compound known to act as a sunscreen in addition to those components discussed herein. As would be apparent to the skilled artisan, compositions used in the practice of the subject invention, can have any combination of the components discussed herein.

While the present invention has been described in terms of various preferred embodiments, those of ordinary skill in the art will appreciate that various modifications, substitutions, omissions, and changes may be made without departed from the spirit of the invention. Accordingly, it is intended that the scope of the present invention not be limited solely by the instant disclosure and the scope of the following claims, including equivalents thereof.

### Features of Parent Application

1. A method of treating cutaneous flushing in humans caused by abnormal, endogenously-induced vasomotor instability comprising administering, to said human via topical dermatological application, a composition comprising at least one selective α₂ adrenergic receptor agonist admixed with a dermatologically acceptable carrier, in an amount effective to reduce, inhibit, reverse or prevent cutaneous facial flushing.
2. The method of feature 1, wherein the composition contains at least one (2-imidazolin-2-ylamino) quinoxaline derivative.
3. The method of feature 1, wherein the cutaneous flushing is facial flushing and the flushing reaction is caused by acne rosacea.
4. The method of feature 2, wherein the cutaneous flushing is facial flushing and the flushing reaction is caused by acne rosacea.
5. The method of feature 1, wherein the cutaneous flushing is facial flushing and the flushing reaction is caused by menopause-associated hot flashes.
6. The method of feature 2, wherein the cutaneous flushing is facial flushing and the flushing reaction is caused by menopause-associated hot flashes.
7. The method of feature 1, wherein the cutaneous flushing is facial flushing and the flushing reaction is the result of hot flashes following orchiectomy.
8. The method of feature 2, wherein the cutaneous flushing is facial flushing and the flushing reaction is the result of hot flashes following orchiectomy.
9. The method of feature 1, wherein the cutaneous flushing is facial flushing and the flushing reaction is caused by ingestion of a substance capable of inducing cutaneous facial flushing selected from the group consisting of alcohol, chocolate, spice, flavor-enhancing additives and mono-sodium glutamate.
10. The method of feature 2, wherein the cutaneous flushing is facial flushing and the flushing reaction is caused by ingestion of a substance capable of inducing cutaneous facial flushing selected from the group consisting of alcohol, chocolate, spice, flavor-enhancing additives and mono-sodium glutamate.
11. The method of feature 2, wherein the composition further comprises an agent, or combination of agents, selected from the group consisting of antibacterial agents, anthelmintic agents, antioxidant agents, steroidal anti-inflammatory agents, non-steroidal anti-inflammatory agents, antiangiogenic agents, and derivatives of retinoic acid.
12. The method of feature 1, wherein the composition further comprises an agent, or combination of agents, selected from the group consisting of antibacterial agents, anthelmintic agents, antioxidant agents, steroidal anti-inflammatory agents, non-steroidal anti-inflammatory agents, antiangiogenic agents, and derivatives of retinoic acid,.
13. The method according to feature 2, wherein said at least one (2-imidazolin-2-ylamino) quinoxaline derivative is brimonidine tartrate.
14. The method of feature 2, wherein the composition further comprises: aloe; compounds that act as sunscreens; or a combination of aloe and compounds that act as sunscreens.
15. The method of feature 2, wherein the composition further comprises a preservative.
16. The method of feature 2, wherein the composition further comprises a halogen.
17. The method of feature 2, wherein the (2-imidazolin-2-ylamino) quinoxaline derivative is combined with an acidic group other than tartrate.
18. The method of feature 1, wherein the composition further comprises an agent, or combination of agents, selected from the group consisting of antibacterial agents, anthelmintic agents, antioxidant agents, steroidal anti-inflammatory agents, non-steroidal anti-inflammatory agents, antiangiogenic agents, and derivatives of retinoic acid.
19. The method of feature 18, wherein the composition further comprises: aloe; compounds that act as sunscreens; or a combination of aloe and compounds that act as sunscreens.
20. The method of feature 19, wherein the composition further comprises a preservative.
21. The method of feature 20, wherein the composition further comprises a halogen.
22. A composition comprising at least one selective α₂ adrenergic receptor agonist admixed with a dermatologically acceptable carrier and one or more agents selected from the group consisting of antibacterial agents, anthelmintic agents, antioxidant agents, steroidal anti-inflammatory agents, non-steroidal anti-inflammatory agents, antiangiogenic agents, derivatives of retinoic acid, aloe, compounds that act as sunscreens, a combination of aloe and compounds that act as sunscreens, preservatives, halogens and combinations of said agents.
23. The composition according to feature 22, wherein the selective α adrenergic receptor agonist is a (2-imidazolin-2-ylamino) quinoxaline derivative.
24. The composition according to feature 23, wherein said (2-imidazolin-2-ylamino) quinoxaline derivative is brimonidine tartrate.
25. The composition according to feature 23, wherein said at least one selective adrenergic receptor agonist is selected from the group consisting of guanabenz, guanfacine, alpha-methyl DOPA (methydopamine), amphetamine, methylphenidate, lofexidine, moxonidine, dexmedetomidine, mivazerol, (2-imidazolin-2-ylamino) quinoxaline derivatives, brimonidine, and combinations thereof.
26. A method for the treatment of flushing in an individual comprising the administration of a composition comprising at least one selective α₂ adrenergic receptor agonist and a carrier in an amount sufficient to prevent, reduce, ameliorate, or inhibit facial flushing.
27. The method of feature 26, wherein said at least one selective adrenergic receptor agonist is selected from the group consisting of guanabenz, guanfacine, alpha-methyl DOPA (methydopamine), amphetamine, methylphenidate, lofexidine, moxonidine, dexmedetomidine, mivazerol, (2-imidazolin-2-ylamino) quinoxaline derivatives, brimonidine, and combinations thereof.
28. The method of feature 1, wherein said at least one selective adrenergic receptor agonist is selected from the group consisting of guanabenz, guanfacine, alpha-methyl DOPA (methydopamine), amphetamine, methylphenidate, lofexidine, moxonidine, dexmedetomidine, mivazerol, (2-imidazolin-2-ylamino) quinoxaline derivatives, brimonidine, and combinations thereof.

## Claims

1. Use of a composition comprising:
at least one selective α₂ adrenergic receptor agonist selected from the group consisting of guanabenz, guanfacine, alpha-methyl DOPA (methydopamine), amphetamine, methylphenidate, lofexidine, moxonidine, dexmedetomidine, mivazerol, and (2-imidazolin-2-ylamino) quinoxaline derivatives, which derivates are formed with an acidic group other than tartrate,
admixed with a dermatologically acceptable carrier
for the manufacture of a medicament for treating cutaneous flushing in humans caused by abnormal, endogenously-induced vasomotor instability, whereby the composition is administered to said human via topical dermatological application in an amount effective to reduce, inhibit, reverse or prevent cutaneous flushing.

2. A composition comprising:
at least one selective α₂ adrenergic receptor agonist selected from the group consisting of guanabenz, guanfacine, alpha-methyl DOPA (methydopamine), amphetamine, methylphenidate, lofexidine, moxonidine, dexmedetomidine, mivazerol, and (2-imidazolin-2-ylamino) quinoxaline derivatives, which derivates are formed with an acidic group other than tartrate, and
a dermatologically acceptable carrier
for use in treating cutaneous flushing in an individual, wherein the composition is to be administered to said individual via topical dermatological application in an amount sufficient to prevent, reduce, ameliorate or inhibit, cutaneous flushing.

3. The use or composition of claim 1 or claim 2, wherein the cutaneous flushing is facial flushing and the flushing reaction is caused by acne rosacea.

4. The use or composition of claim 1 or claim 2, wherein the cutaneous flushing is facial flushing and the flushing reaction is caused by menopause-associated hot flashes.

5. The use or composition of claim 1 or claim 2, wherein the cutaneous flushing is facial flushing and the flushing reaction is the result of hot flashes following orchiectomy.

6. The use or composition of claim 1 or claim 2, wherein the cutaneous flushing is facial flushing and the flushing reaction is caused by ingestion of a substance capable of inducing cutaneous facial flushing selected from the group consisting of alcohol, chocolate, spice, flavor-enhancing additives and mono-sodium glutamate.

7. The use or composition for use according to claim 1 or claim 2, wherein the composition further comprises an agent, or combination of agents, selected from the group consisting of antibacterial agents, anthelmintic agents, antioxidant agents, steroidal anti-inflammatory agents, non-steroidal anti-inflammatory agents, antiangiogenic agents, and derivatives of retinoic acid.

8. The use or composition for use according to claim 1, claim 2, or claim 7,wherein the composition further comprises: aloe; compounds that act as suncreens; or a combination of aloe and compounds that act as sunscreens.

9. The use or composition for use according to claim 1, claim 2, or claim 7, wherein the composition further comprises a preservative.

10. The use or composition for use according to claim 1, claim 2, or claim 7, wherein the composition further comprises a halogen.

11. A composition comprising:
at least one selective α₂ adrenergic receptor agonist selected from the group consisting of guanabenz, guanfacine, alpha-methyl DOPA (methydopamine), amphetamine, methylphenidate, lofexidine, moxonidine, dexmedetomidine, mivazerol, and (2-imidazolin-2-ylamino) quinoxaline derivatives, which derivates are formed with an acidic group other than tartrate,
admixed with a dermatologically acceptable carrier and one or more agents selected from the group consisting of antibacterial agents, anthelmintic agents, antioxidant agents, steroidal anti-inflammatory agents, non-steroidal anti-inflammatory agents, antiangiogenic agents,
derivatives of retinoic acid, aloe, compounds that act as sunscreens, a combination of aloe and compounds that act as sunscreens, preservatives, and halogens.
